# EUROPÄISCHE PATENTSCHRIFT

(11) **EP 0 809 435 B1**
(45) Veröffentlichungstag und Bekanntmachung des Hinweises auf die Patenterteilung: **12.05.1999**
(21) Anmeldenummer: 96901342.4
(22) Anmeldetag: 01.02.1996
(51) Int. Cl.: A01N 35/02, C02F 1/50

(54) **ZUR ACROLEINFREISETZUNG BEFÄHIGTE ZUSAMMENSETZUNG UND DEREN VERWENDUNG**
COMPOSITION CAPABLE OF RELEASING ACRALDEHYDE AND ITS USE
COMPOSITION CAPABLE DE LIBERER DE L'ACROLEINE ET SON UTILISATION

(30) Priorität: 16.02.1995 DE 19505171
(43) Veröffentlichungstag der Anmeldung: 03.12.1997
(73) Patentinhaber: DEGUSSA AG, 60311 Frankfurt (DE)
(72) Erfinder: WERLE, Peter, D-63571 Gelnhausen (DE); TRAGESER, Martin, D-63571 Gelnhausen (DE); HELMLING, Oswald, D-63594 Hasselroth (DE); JAKOB, Harald, D-63594 Hasselroth (DE)
(86) Internationale Anmeldenummer: EP9600415
(87) Internationale Veröffentlichungsnummer: WO9625040

(56) Entgegenhaltungen:
- EP-A- 0 066 224
- EP-A- 0 493 658
- DE-A- 4 326 575
- GB-A- 2 079 153
- US-A- 3 690 857
- US-A- 4 851 583
- CHEMICAL ABSTRACTS, vol. 73, no. 22, 30.November 1970 Columbus, Ohio, US; abstract no. 110169, T.OUCHI ET AL.: "Polymerization of diallylidenepentaerythritol. 7. Copolymerization of 2-vinyl-1,3-dioxolane with maleic anhydride" XP002004740 & KOGYO KAGAKU ZASSHI, Bd. 73, Nr. 7, 1970, Seiten 1717-1719,
- CHEMICAL ABSTRACTS, vol. 74, no. 22, 31.Mai 1971 Columbus, Ohio, US; abstract no. 112779, XP002004741 & JP,A,45 040 054 (SHOWA) 16.Dezember 1970
- CHEMICAL ABSTRACTS, vol. 70, no. 2, 13.Januar 1969 Columbus, Ohio, US; abstract no. 4893, Z.JEDLINSKI ET AL.: "Polymerization of cyclic acetals. I. Polymerization of 2-vinyl-1,3-dioxane and 2-isopropenyl-1,3-dioxane" XP002004742 & J.POLYM.SCI., PART A-1, Bd. 6, Nr. 11, 1968, Seiten 3182-3184,

## Beschreibung

Die Erfindung richtet sich auf eine zur Acroleinfreisetzung befähigte Zusammensetzung, welche durch Freisetzung von Acrolein in Gegenwart von Wasser eine biozide Wirksamkeit aufweist. Die Erfindung richtet sich auch auf die Verwendung der Zusammensetzung zur Bekämpfung mikrobieller, pflanzlicher und tierischer Schädlinge Sowie ein Verfahren zur Durchführung.

In klimatisch warmen Zonen mit ausgedehnten landwirtschaftlich genutzten Feldern und Plantagen wird ein großes Netz an Bewässerungskanälen unterhalten. In diesen Kanälen kommt es leicht zur Veralgung und Verkrautung durch Wasserpflanzen. Die Pflanzen behindern die Fließgeschwindigkeit in den Kanälen und gefährden die störungsfreie Funktion der zum Betrieb notwendigen Pumpstationen. Aus diesem Grund ist es üblich, das Wasser in einem derartigen Bewässerungssystem mit einem Biozid zu dotieren. In der Praxis hat sich als Biozid Acrolein bewährt.

Acrolein bietet neben der bislang unübertroffenen Wirksamkeit auch den Vorteil, daß es in Wasser nach kurzer Zeit abgebaut ist. Damit ist Acrolein bis zur Bewässerung der Felder nicht mehr als Biozid wirksam, sondern zu Pflanzenphysiologisch unbedenklichen Folgeprodukten abgebaut. Aufgrund der physikalischen und chemischen Eigenschaften des Acroleins ist aber seine Handhabung risikoreich: Acrolein ist giftig, stark inhalationstoxisch, stechend und tränenreizend und leicht entzündlich (Flammpunkt -29 °C, Siedepunkt 53 °C); durch Kontamination des Acroleins mit Verunreinigungen besteht ferner explosionsartige Polymerisationsgefahr.

In Anbetracht der dargestellten Risiken, welche sich sowohl beim Transport als auch bei der Handhabung von Acrolein ergeben, besteht ein Bedarf, die Dotierung mit einem vergleichbar wirksamen Biozid vorzunehmen, dessen Handhabung aber wesentlich weniger risikoreich ist.

Aus der US-PS 4,851,583 ist bekannt, das als Pestizid wirksame Acrolein durch Deacetalisierung von Acroleinacetalen, darunter auch cyclischen Acroleinacetalen, in Gegenwart eines stark sauren Ionenaustauschers als Katalysator zu gewinnen. Zur direkten Dotierung strömender Gewässer ist dieses Verfahren aber nicht geeignet, weil es rasch zur Verschmutzung beziehungsweise Inaktivierung des Ionenaustauschers käme. Die meisten Acroleinacetale weisen ferner nur eine sehr begrenzte Löslichkeit in Wasser auf, und zudem ist die Lösegeschwindigkeit niedrig: Beispielsweise werden zur Herstellung einer unter Praxisbedingungen nahezu gesättigten Lösung von 2-Vinyl-1,3-dioxolan (VDL) in Wasser (etwa 8 Gew.-%) bei intensiver Durchmischung etwa 10 Minuten benötigt; Die genannten Faktoren erschwerten bisher die Verwendung von Acroleinacetalen zur Freisetzung von Acrolein am Ort des Bedarfs, also unmittelbar an den Bewässerungskanälen, zumal dort elektrische Energie zwecks Intensivmischung nicht zur Verfügung steht.

Ein anderer Weg zur Dotierung von Wasser wird in der US-PS 5,183,944 beschrieben: Hiernach wird Acrolein durch Deacetalisierung von Acroleinacetalen in wäßriger Phase in Gegenwart eines stark sauren Katalysators gebildet. Zum Dotieren wäßriger Lösungen wird das während der Deacetalisierung in einem Reaktionsgefäß gebildete Acrolein ständig aus der wäßrigen Phase entfernt und in die zu dotierende wäßrige Lösung überführt wird. Die genannte Überführung erfolgt entweder mittels eines durch das Reaktionsgefäß geführten Gasstromes, der anschließend in die zu dotierende wäßrige Lösung geleitet wird, oder unter Verwendung einer Flüssigkeitsstrahlpumpe, deren Treibmittel die zu dotierende wäßrige Lösung ist. Dieses Verfahren eignet sich zwar zum Dotieren von beispielsweise Kühlwasserkreisläufen in Industrieanlagen, es läßt sich aber nicht zur Dotierung des Wassers in Bewässerungskanälen verwenden, weil am Ort der Dotierung im allgemeinen weder elektrische Energie zum Betreiben der Pumpen noch chemisch geschultes Fachpersonal zur Verfügung stehen.

In der noch nicht veröffentlichten DE-Patentanmeldung 43 26 575 wird ein Verfahren zum Dotieren strömender Gewässer mit Acrolein gelehrt, das die Nachteile der direkten Verwendung von Acrolein vermeidet und einfacher durchführbar ist als vorbekannte Verfahren unter Verwendung eines Acroleinacetals als Biozid-Percursor. Bei diesem Verfahren werden eine 25 bis 95 gew.-%ige Lösung eines Acroleinacetals in einem organischen Lösungsmittel und eine 3 bis 30 gew.-%ige wäßrige Mineralsäurelösung zunächst in festgelegtem Mengenverhältnis in eine Mischkammer eingebracht, das Reaktionsgemisch anschließend durch einen speziell konstruierten Deacetalisierungsreaktor geleitet und dann in das zu dotierende Gewässer überführt.

Obwohl das zuvor gewürdigte Verfahren eine Problemlösung darstellt, weist es doch noch einige Nachteile auf: Zur Durchführung sind ein spezieller Deacetalisierungsreaktor und außer einem druckfesten Vorratsbehälter für das Acroleinacetal zusätzlich ein solcher für eine wäßrige Mineralsäure erforderlich, wodurch sich der Aufwand erhöht; auch die Verhältnisregelung zwischen dem Acroleinacetal und der wäßrigen Säure mittels Lochblenden ist nicht unproblematisch.

Die noch nicht veröffentliche DE-Patentanmeldung P 44 41 315.7 lehrt eine zur Acroleinfreisetzung befähigte und damit zur Biozidbehandlung geeignete wasserfreie Zusammensetzung, welche ein Acetal des Acroleins mit einem Alkohol mit 1 bis 6 C-Atomen und 1 bis 4 Hydroxylgruppen und eine im Acetal zumindest teilweise lösliche und damit chemisch verträgliche Säure mit einem pKₛ-Wert von kleiner 4 enthält. Sofern die Säure nicht absolut wasserfrei ist, kann es allerdings nach längerer Lagerzeit zu einer Verfärbung und einem unerwünschten Viskositätsanstieg der Zusammensetzung kommen.

Die Aufgabe der vorliegenden Erfindung besteht darin, ein weiteres Verfahren aufzuzeigen, womit mikrobielle, pflanzliche und tierische Schädlinge mittels Acrolein bekämpft werden können, ohne jedoch Acrolein selbst an den Bedarfsort transportieren und dort handhaben zu müssen. Das Verfahren sollte einfach handhabbar und nicht auf elektrische Energiezufuhr, etwa zum Betreiben von Pumpen, Rühr- und Heizvorrichtungen, angewiesen sein. Eine weitere Aufgabe richtet sich auch darauf, eine sicher handhabbare, zur Acroleinfreisetzung befähigte Zusammensetzung zur Verfügung zu stellen, bei deren Verwendung sich die Bereitstellung einer zweiten Chemikalie und deren Zumischung zur Zusammensetzung am Ort der Verwendung zwecks Acroleinfreisetzung erübrigen. Schließlich sollte sich die Freisetzung von Acrolein aus der Zusammensetzung ohne das Erfordernis eines speziellen Reaktors durchfuhren lassen.

Aus Chemical Abstracts 73 (1970) No. 110169 und 74 (1971) No. 112779 ist bekannt, 2-Vinyl-1,3-dioxolan beziehungsweise 2-Vinyl-1,3-dioxan mit Maleinsäureanhydrid in Gegenwart eines Peroxidinitiators zu copolymerisieren. Die kationische Polymerisation von 2-Vinyl-1,3-dioxan mittel TiCl₄ oder SnCl₄ ist aus Chemical Abstracts 70 (1969) Nr. 4893 bekannt.

Gelöst wird die Aufgabe durch ein Mittel zur Bekämpfung mikrobieller, pflanzlicher und tierischer Schädlinge auf der Basis einer beim Kontaktieren mit Wasser Acrolein freisetzenden Zusammensetzung, enthaltend ein Acroleinacetal, eine durch Hydrolyse eine Säure mit einem pKₛ-Wert von kleiner 4 freisetzende Verbindung und ein Tensid.

Die Unteransprüche richten sich auf bevorzugte Ausführungsformen der Mittel.

Es wurde gefunden, daß ein Gemisch aus einem oder mehreren Acroleinacetalen und einer oder mehreren anspruchsgemäßen hydrolysierbaren säurebildenden Verbindungen und einem Tensid in Abwesenheit von Wasser ausreichend lagerstabil ist, es also zu keiner Deacetalisierung kommt. Das erfindungsgemäße Mittel ist damit in feuchtigkeitsdichten Gebinden transport- und lagerfähig und weist nicht die vom Acrolein bekannten Handhabungsprobleme auf. Erst beim Kontaktieren der Zusammensetzung mit Wasser kommt es zur raschen Hydrolyse der saurebildenden Verbindung und in deren Folge zur Deacetalisierung des Acetals und Freisetzung des biozid wirksamen Acroleins.

Bei dem erfindungsgemäß in der Zusammensetzung enthaltenen Acroleinacetal handelt es sich um ein offenkettiges oder cyclisches Acetal, das keine freien Hydroxylgruppen aufweist, wobei die zugrundeliegende Alkoholkomponente im allgemeinen auf einem ein- oder zweiwertigen Alkohol mit 1 bis 6 C-Atomen basiert. Als einwertige Alkoholkomponente des Acetals sind insbesondere Methanol, Ethanol, n- und iso-Propanol, als zweiwertige Alkoholkomponente insbesondere Ethylenglykol, 1,2- und 1,3-Propylenglykol zu nennen. Im Prinzip kann das Acetal auch auf einer drei- oder vierwertigen Alkoholkomponente, wie Glycerin, Trimethylolpropan, 1,2,6-Hexantriol und Pentaerythrit basieren, jedoch lassen sich solche Acetale nur schwer hydroxylgruppenfrei erzeugen. Besonders bevorzugte Acroleinacetale sind solche mit einer 2-Vinyl-1,3-dioxolan- oder 2-Vinyl-1,3-dioxanstruktur, insbesondere 2-Vinyl-1,3-dioxolan und 2-Vinyl-1,3-dioxan, welche leicht erhältlich sind aus Acrolein und Ethylenglykol beziehungsweise 1,3-Propandiol. Acroleinacetale mit längeren aliphatischen Gruppen in der Alkoholkomponente werden wegen ihrer geringen Löslichkeit in Wasser weniger bevorzugt.

An die in der Zusammensetzung enthaltene, bei der Hydrolyse säurebildende Verbindung werden eine Reihe von Anforderungen gestellt: Sie muß in ausreichender Menge im Acroleinacetal löslich oder homogen darin emulgiert oder suspendiert sein. Bei der Hydrolyse der säurefreisetzenden Verbindung können eine oder mehrere Säuren mit einem pKₛ von kleiner 4 resultieren. Mindestens eine der bei der Hydrolyse freigesetzen Säuren muß einen pKₛ-Wert von kleiner 4, vorzugsweise kleiner 2,5, insbesondere kleiner 2, aufweisen. Besonders bevorzugt wird eine hydrolysierbare Verbindung, aus welcher bei der Hydrolyse Chlorwasserstoff entsteht. Die säurebildende Verbindung und das Acroleinacetal müssen in Abwesenheit von Feuchtigkeit im Gemisch miteinander chemisch beständig sein - diese Beständigkeit wird der Fachmann durch orientierende Versuche ermitteln, um zu einer lagerstabilen erfindungsgemäßen Zusammensetzung zu gelangen. Vorzugsweise sollen die säurebildende Verbindung sowie deren Hydrolyseprodukte auch umweltverträglich, also biologisch abbaubar und möglichst nicht toxisch sein.

Unter den unter Bildung einer Säure hydrolysierbaren Verbindungen sind organische Säureanhydride unter der Prämisse gut geeignet, daß der pKₛ-Wert der Säure unter 4 liegt. Beispielhaft sind zu nennen: Maleinsäureanhydrid sowie 2-mono- und 2,3-disubstituiertes Maleinsäureanhydrid, wobei der/die Substituent(en) vorzugsweise elektronenanziehend sind, ferner Pyromellitsäuremono- oder bevorzugt -dianhydrid.

Eine weitere Klasse erfindungsgemäß verwendbarer hydrolysierbarer säurebildender Verbindungen sind organische Säurehalogenide, insbesondere Chloride, Bromide und Jodide, wobei Säurechloride bevorzugt sind. Bei der dem Säurehalogenid zugrundeliegenden organischen Säure kann es sich um eine aliphatische, cycloaliphatische, aromatische oder heterocyclische Säure handeln, beispielsweise um eine Mono-, Di-, Tri- oder Polycarbonsäure, eine Sulfonsäure oder Phosphonsäure. Üblicherweise werden solche Säurehalogenide verwendet, deren organischer Rest 1 bis 12 C-Atome, vorzugsweise 2 bis 6 C-Atome, enthält. Beispielhaft genannt werden: Acetylchlorid, Acetylbromid, Acetyljodid, Propionylchlorid, Oxalylchlorid, Succinylchlorid, Adipoylchlorid, Benzoylchlorid.

Eine besonders wirksame Klasse hydrolysierbarer Verbindungen sind anorganische, bei der Hydrolyse Halogenwasserstoff, insbesondere HCl, freisetzende Halogenverbindungen. Zweckmäßigerweise fallen diese Verbindungen unter die allgemeinen Formeln MXₘ oder MOX₍ₘ₋₂₎ oder RpMX₍ₘ₋ₚ₎, wobei M für Silicium, Titan, Zinn, Phosphor, Arsen, Antimon oder Schwefel, R für Methyl oder Ethyl oder Phenyl und X für Cl, Br oder J, insbesondere Cl, und m für die Wertigkeit von M steht; m ist eine ganze Zahl zwischen 3 und 5, p eine ganze Zahl zwischen 1 und 3 und (m-p) ungleich 0. Auch Oligomere der genannten Stoffklassen, wie etwa (Xₘ₋₁M)₂O, können verwendet werden. Besonders bevorzugte Verbindungen sind Siliciumtetrachlorid und Titantetrachlorid sowie Phosphoroxychlorid. Bei SiCl₄ ist aufgrund des günstigen Molekulargewichts und der hohen Säurestärke der gebildeten Salzsäure ein Zusatz von 0,5 bis 5 Gew.-%, insbesondere 1 bis 3 Gew.-%, bezogen auf die Zusammensetzung für eine rasche Hydrolyse des Acroleinacetals völlig ausreichend.

Das Acroleinacetal oder Acroleinacetalgemisch und die säurebildende hydrolysierbare Verbindung oder Gemisch solcher Verbindungen können an sich in beliebigem Molverhältnis in der Zusammensetzung anwesend sein, zweckmäßigerweise liegt aber das Molverhältnis im Bereich von 50 zu 1 bis 1 zu 1, insbesondere 50 zu 1 bis 10 zu 1. Je höher die Konzentration an säurebildender Verbindung in der Zusammensetzung und je höher die Säurestärke der bei der Hydrolyse gebildeten Säure ist, desto rascher erfolgt die Deacetalisierung des Acetals nach Kontaktieren der Zusammensetzung mit Wasser. Der Gehalt an Acroleinacetal in der Zusammensetzung wird, um mit einer gegebenen Menge Zusammensetzung eine möglichst hohe biozide Wirksamkeit erzielen zu können, möglichst hoch sein. Eine besonders bevorzugte Zusammensetzung enthält ein bevorzugtes Acroleinacetal oder Gemisch mehrerer Acetale und eine oder mehrere bevorzugte säurebildende Verbindungen aus der Reihe der Säureanhydride, Säurehalogenide und anorganischen Halogenverbindungen im Gewichtsverhältnis im Bereich von 99,5 zu 0,5 bis 80 zu 20, inbesondere im Bereich von 98 zu 2 bis 90 zu 10.

Das erfindungsgemäße Mittel enthält zusätzlich ein oder mehrere, in Gegenwart der saurebildenden Verbindung und des Acroleinacetals stabile Tenside. Die Einsatzmenge an Tensid liegt üblicherweise zwischen 5 und 0,05 Gew.-%, bezogen auf die Zusammensetzung, meistens ist aber eine Menge zwischen 0,2 bis 1 Gew.-% ausreichend. Besondes geeignete Tenside sind Ethoxylierungsprodukte von Fettalkoholen, Alkylphenolen, Fettsäuren, Fettamiden und Glycerinmono- und -difettsäureestern, welche hydroxylgruppenfrei sind oder nur eine geringe Hydroxylzahl aufweisen. Das Tensid muß in der Zusammensetzung ausreichend lagerstabil und löslich und zudem in der Lage sein, die Zusammensetzung nach Kontaktieren mit Wasser sicher zu emulgieren. In Abwesenheit eines Tensids oder zu geringer Konzentration hieran kann es bei wenig wasserlöslichen Acroleinacetalen, etwa 2-Vinyl-1,3-dioxolan, zu einer Entmischung kommen, so daß die Deacetalisierung unvollständig oder verzögert abläuft. In Anwesenheit einer ausreichenden Menge eines wirksamen Tensids löst sich die erfindungsgemäße Zusammensetzung innerhalb weniger Sekunden klar auf, und die Deacetalisierung läuft auch bei etwa 20 °C innerhalb weniger Minuten praktisch quantitativ ab.

Außer den erfindungswesentlichen Komponenten kann das Mittel weitere Zusatzstoffe enthalten, soweit sie in der Zusammensetzung stabil sind. Mögliche weitere Zusatzstoffe sind beispielsweise andere Biozide und wasserlösliche aprotische organische Lösungsmittel.

Die erfindungsgemäßen Mittel lassen sich durch einfaches Zusammenmischen der Komponenten herstellen und sind im allgemeinen klare Lösungen. Die Verträglichkeit der Komponenten untereinander wird zuvor durch Lagertests sichergestellt. Das Mischen und Lagern der Zusammensetzung erfolgt unter Ausschluß von Feuchtigkeit.

Die erfindungsgemäßen Mittel lassen sich, da beim Kontakt mit Wasser das hochwirksame Biozid Acrolein freigesetzt wird, zur Bekämpfung mikrobieller, pflanzlicher und tierischer Schädlinge verwenden. Unter den Schädlingen sind insbesondere Bakterien und Pilze, Algen und Wasserkräuter, Insekten, Würmer und Nager zu verstehen.

Zur Bekämpfung mikrobieller, pflanzlicher und tierischer Schädlinge mittels Acrolein wird ein erfindungsgemäßes Mittel entweder (i) unmittelbar mit einem die zu bekämpfenden Schädlinge enthaltenden feuchten Medium in Kontakt gebracht oder (ii) im Gewichtsverhältnis von 1 zu 1 bis 1 zu 50, vorzugsweise 1 zu 5 bis 1 zu 20, mit Wasser gemischt, das Reaktionsgemisch nach einer zur weitgehenden Deacetalisierung des in der Zusammensetzung enthaltenden Acroleinacetals erforderlichen Reaktionszeit unmittelbar oder nach weiterer Verdünnung mit Wasser in das die zu bekämpfenden Schädlinge enthaltende Medium eingebracht.

Das Verhältnis Acroleinacetal zu Wasser in der Verfahrensalternative (ii) wird zweckmäßigerweise so gewählt, daß während der Deacetalisierung ein pH-Wert um/unter 2 während einiger Minuten aufrechterhalten und erst anschließend Weiter verdünnt wird.

Zum Zwecke einer raschen Auflösung der Zusammensetzung in Wasser wird diese vorzugsweise mit einer Ein- oder besser Mehrfachsprühdüse in Wasser eingedüst, so daß sofort feinste und damit gut emulgierbare und rasch lösliche Tröpfchen resultieren. Das Eindüsen läßt sich beispielsweise unter Verwendung entsprechender Düsen und einer Druckgasflasche bewirken. Bei der Verfahrensalternative (i) wird Acrolein dem Feuchtegehalt des Mediums entsprechend langsamer freigesetzt. Die Verfahrensalternative (i) läßt sich beispielsweise zur Bekämpfung von Nematoden oder Nagern im Erdboden anwenden.

Zur Bekämpfung von aquatischen Schädlingen, etwa Algen und Kräutern in Bewässerungskanälen, eignet sich besonders die Alternative (ii). Hierbei wird das deacetalisierte Gemisch kontinuierlich oder periodisch in einer solchen Menge in das die Schädlinge enthaltende Wasser eingeleitet, daß darin ein Acroleingehalt im Bereich von 0,1 bis 20 ppm Acrolein aufrechterhalten wird.

Vorteile der erfindungsgemäßen Mittel sind ihre leichte Herstellbarkeit, ihre gute Lager- und Handhabbarkeit und ihre unmittelbare Anwendbarkeit zur Bekämpfung von Schädlingen. Da die Mittel selbst eine Vorstufe des Deacetalisierungskatalysators enthalten, muß ein solcher nicht am Ort der Verwendung einem Acroleinacetal in dosierter Form zugemischt werden. Aufgrund der ausgewählten Bestandteile der Zusammensetzung sind weder eine aufwendige Vorrichtung noch ein spezieller Deacetalisierungsreaktor erforderlich, um aus dem Acroleinacetal Acrolein freisetzen zu können. Das Verfahren zur Bekämpfung von bakteriellen, pflanzlichen und tierischen Schädlingen gestaltet sich damit einfach, erfordert keine elektrische Energie und kann von wenig geschultem Personal durchgeführt werden.

### Beispiel 1

In einer Mischung aus 1417,5 g Vinyl-1,3-dioxolan (VDL) und 7,5 g Tensid auf der Basis eines Polyoxyethylen-glycerin-monooleats (Tegotens® 02 der Firma Th. Goldschmidt AG) wurden unter Rühren 75,0 g Maleinsäureanhydrid gelöst.
a) 100 ml dieser Lösung wurden in 600 ml Wasser eingetragen; nach 6 min wurde die Acroleinfreisetzung durch Neutralisierung auf pH 6 gestoppt: Die Umsetzung des VDL betrug 97,4 % (gaschromatographisch ermittelt).
   Der pKₛ-Wert der aus dem Anhydrid gebildeten und die Deacetalisierung katalysierenden Maleinsäure beträgt etwa 1,8.
b) 100 g einer Zusammensetzung gemäß Beispiel 1 wurden mittels einer Sprühdüse mit einem Druck aus einer N₂-Druckgasflasche von 3 bar in 1000 ml Wasser eingedüst. Die zunächst gebildete Emulsion wurde in wenigen Sekunden eine klare Lösung. Nach 10 Minuten Verweilzeit hatten sich 99,6 % des im VDL gebundenen Acroleins freigesetzt (gaschromatographische Bestimmung).

Durch Zugabe des freigesetztes Acrolein enthaltenden wäßrigen Konzentrats zu aquatische Schädlinge enthaltendem Wasser im Verhältis von beispielsweise 1 zu 5000 stellt sich eine Acroleinkonzentration von etwa 10,6 ppm (parts per million) ein, welche zur Bekämpfung der Schädlinge geeignet ist.

### Beispiel 2

Zu einer Mischung aus 282,1 g VDL und 1,45 g Tensid (Tegotens® 02) wurden unter Rühren 7,5 g Essigsäurechlorid gegeben. Man erhielt sofort eine homogene Lösung. 16,2 ml dieser Lösung wurden unter Rühren in 162 ml Wasser gegeben. Die Reaktion wurde nach 6 min gestoppt. Die gaschromatographische Analyse ergab eine Umsetzung von 99,7 % des eingesetzten VDL.

### Beispiel 3

100 g VDL wurden mit 2,0 g Siliciumtetrachlorid gemischt. Die Mischung ist homogen und lagerstabil.

Unter Rühren trug man diese Mischung in 600 ml Wasser ein und stoppte die Reaktion nach 5 min durch Natronlaugezugabe. Die Umsetzungsrate betrug 99,5 %.

### Beispiel 4: Verfahren zur Verhinderung einer Veralgung und Verkrautung strömender Gewässer

Die erfindungsgemäße Zusammensetzung gemäß Beispiel 1 wird in einem mit Steigrohr versehenen feuchtigkeitsdicht verschlossenen Behälter zum Applikationsort gebracht. Durch Anlegen eines Drucks von 3 bar aus einer N₂-Druckgasflasche auf den Behälter wird die aus dem Steigrohr austretende Zusammensetzung über einen Schlauch in das zu dotierende Wasser geleitet. Um die für die Acetalspaltung notwendigen Bedingungen hinsichtlich Zeit und Acidität zu erhalten, wird die sich bildende Lösung zunächst nur soweit verdünnt, daß über einen Zeitraum von etwa 5 bis 10 Minuten ein pH-Wert von etwa 1,5 bis 2 aufrechterhalten wird. Dies erfolgt beispielsweise in einem spiralförmig gerollten Schlauch. Nach ausreichender Deacetalisierung wird das Acrolein enthaltende deacetalisierte wäßrige Konzentrat mit einer ausreichenden Menge des zu dotierenden Wassers verdünnt, um zur gewünschten Anwendungskonzentration zu gelangen.

Eine weitere geeignete Maßnahme zur Einhaltung optimaler Deacetalisierungsbedingungen besteht darin, daß die erfindungsgemäße Zusammensetzung in einen mit einer definierten Menge Wasser gefüllten Behälter eingedüst wird; dieser Behälter befindet sich zweckmäßigerweise selber in dem zu dotierenden Wasser. Nach Ablauf der Verweilzeit wird der Behälter geöffnet und das freigesetzte Acrolein in den Kanal geschwemmt.

### Beispiel 5

In einer Mischung aus 95 g Acroleindimethylacetal (ADMA) und 0,5 Tensid (Tegotens®02) werden unter Rühren 5 g Maleinsäureanhydrid gelöst. Diese Lösung wird in 600 ml Wasser eingetragen. Das schwer lösliche Acetal wird rasch emulgiert unter Bildung einer milchig trüben Emulsion. Nach 10 min. ist die Lösung klar und homogen. Nach Abstoppen der Reaktion werden noch 0,5 % ADMA gefunden, d. h., der Umsetzungsgrad beträgt 99,5 %.

### Beispiel 6

In einer Mischung aus 142,5 g 2-Vinyldioxan und 1,0 g eines Emulgators auf Basis Nonylphenolethoxylat werden 7,5 g Maleinsäureanhydrid gelöst. Die klare Lösung wird unter Rühren in 900 ml Wasser eingetragen. Das Vinyldioxan wird emulgiert und langsam gelöst und ist nach 10 bis 15 min. vollständig hydrolysiert. Die Lösung ist nun klar und farblos und riecht stark nach Acrolein. Gaschromatographisch wird der Umsatz zu 99,2 % gefunden.

## Patentansprüche

1. Mittel zur Bekämpfung mikrobieller, pflanzlicher und tierischer Schädlinge auf der Basis einer beim Kontaktieren mit Wasser Acrolein freisetzenden Zusammensetzung, enthaltend ein Acroleinacetal, eine durch Hydrolyse eine Säure mit einem pKₛ-Wert von kleiner 4 freisetzende Verbindung und ein Tensid.

2. Mittel nach Anspruch 1,
dadurch gekennzeichnet,
daß es als säurefreisetzende Verbindung ein organisches Sätireanhydrid, organisches Säurehalogenid oder eine anorganische, bei der Hydrolyse Halogenwasserstoff bildende Halogenverbindung enthält.

3. Mittel nach Anspruch 2,
dadurch gekennzeichnet,
daß es als säurefreisetzende Verbindung Maleinsäureanhydrid oder Pyromellithsäure-dianhydrid oder ein Säurechlorid einer Carbonsäure, Phosphonsäure oder Sulfonsäure enthält.

4. Mittel nach Anspruch 2,
dadurch gekennzeichnet,
daß es als Halogenwasserstoff bildende Halogenverbindung ein Chlorid oder Oxychlorid von Silicium, Titan, Zinn, Phosphor oder Schwefel enthält.

5. Mittel nach einem der Ansprüche 1 bis 4,
dadurch gekennzeichnet,
daß das Acroleinacetal offenkettig oder cyclisch ist, keine freien Hydroxylgruppen aufweist und auf einer ein- oder zweiwertigen Alkoholkomponente mit 1 bis 6 C-Atomen basiert.

6. Mittel nach Anspruch 5,
dadurch gekennzeichnet,
daß es als Acroleinacetal 2-Vinyl-1,3-dioxolan enthält.

7. Mittel nach einem der Ansprüche 1 bis 6,
dadurch gekennzeichnet,
daß der pKₛ-Wert der freigesetzten Säure kleiner 2,5 ist.

8. Mittel nach einem der Ansprüche 1 bis 7,
dadurch gekennzeichnet,
daß es Acroleinacetal und die säurefreisetzende Verbindung im Molverhältnis von 50 zu 1 bis 10 zu 1 enthält.

9. Mittel nach einem der Ansprüche 1 bis 8,
dadurch gekennzeichnet,
daß es als Tensid ein im wesentlichen hydroxylgruppenfreies oder -armes Ethoxylierungsprodukt eines Fettalkohols, einer Fettsäure, eines Fettamins oder eines Glycerinmono oder -difettsäureesters enthält.

10. Mittel nach einem der Ansprüche 1 bis 9,
dadurch gekennzeichnet,
daß es im wesentlichen 80 bis 98 Gew.-% Acroleinacetal, 2 bis 20 Gew.-% säurefreisetzende Verbindung und bis zu 5 Gew.-% Tensid enthält.

11. Verfahren zur Bekämpfung mikrobieller, pflanzlicher und tierischer Schädlinge mittels Acrolein,
dadurch gekennzeichnet,
daß man ein Mittel gemäß einem der Ansprüche 1 bis 10 entweder unmittelbar mit einem die zu bekämpfenden Schädlinge enthaltenden feuchten Medium in Kontakt bringt oder das Mittel im Gewichtsverhältnis von 1:1 bis 1:50 mit Wasser mischt, das Reaktionsgemisch nach einer zur weitgehenden Deacetalisierung des in der Zusammensetzung enthaltenen Acroleinacetals erforderlichen Reaktionszeit unmittelbar oder nach weiterer Verdünnung mit Wasser in das die zu bekämpfenden Schädlinge enthaltende Medium einbringt.

12. Verfahren nach Anspruch 11 zur Verhinderung einer Veralgung und Verkrautung aquatischer Systeme,
dadurch gekennzeichnet,
daß man in das aquatische System das deacetalisierte Reaktionsgemisch kontinuierlich oder periodisch in einer solchen Menge einleitet, daß darin ein Acroleingehalt im Bereich von 0,1 bis 20 ppm aufrechterhalten wird.

## Claims

1. An agent for controlling microbial, vegetable and animal pests based on a composition which releases acrolein on contact with water, containing an acrolein acetal, a compound which releases an acid with a pKa value of less than 4 on hydrolysis and a surfactant.

2. An agent according to Claim 1,
characterised in that it contains an organic acid anhydride, an organic acid halide or an inorganic halogen compound which forms a hydrogen halide during hydrolysis as an acid-releasing compound.

3. An agent according to Claim 2,
characterised in that it contains maleic anhydride or pyromellitic dianhydride or an acid chloride of a carboxylic acid, phosphonic acid or sulfonic acid as an acid-releasing compound.

4. An agent according to Claim 2,
characterised in that it contains a chloride or oxychloride of silicon, titanium, tin, phosphorus or sulfur as a halogen compound which forms a hydrogen halide.

5. An agent according to one of Claims 1 to 4,
characterised in that the acrolein acetal has an open-chain or cyclic structure, contains no free hydroxyl groups and is based on a monohydric or dihydric alcohol component with 1 to 6 carbon atoms.

6. An agent according to Claim 6,
characterised in that it contains 2-vinyl-1,3-dioxolane as an acrolein acetal.

7. An agent according to one of Claims 1 to 6,
characterised in that the pKa value of the acid released is less than 2.5.

8. An agent according to one of Claims 1 to 7,
characterised in that it contains acrolein acetal and the acid-releasing compound in the molar ratio 50:1 to 10:1.

9. An agent according to one of Claims 1 to 8,
characterised in that it contains, as surfactant, the ethoxylation product of a fatty alcohol, a fatty acid, a fatty amine or an ester of a glycerine mono- or di-fatty acid, said product containing substantially no or very few, hydroxyl groups.

10. An agent according to one of Claims 1 to 9,
characterised in that it contains substantially 80 to 98 wt.% of acrolein acetal, 2 to 20 wt.% of acid-releasing compound and up to 5 wt.% of surfactant.

11. A process for controlling microbial, vegetable and animal pests by means of acrolein,
characterised in that an agent according to one of Claims 1 to 10 is either placed directly in contact with a moist medium containing the pest to be controlled or the agent is mixed with water in the ratio by weight of 1:1 to 1:50 and the reaction mixture is introduced directly, or after further dilution with water, into the medium containing the pest to be controlled after a reaction time required for extensive deacetalisation of the acrolein acetal which is contained in the composition.

12. A process according to Claim 11 for preventing the growth of algae and plants in aquatic systems,
characterised in that the deacetalised reaction mixture is introduced continuously or periodically in an amount such that an acrolein concentration in the range 0.1 to 20 ppm is maintained therein.

## Revendications

1. Agent pour lutter contre les nuisances microbiennes, végétales et animales sur base d'une composition libérant de l'acroléine par un contact avec de l'eau, contenant un acétal d'acroléine, un composé libérant par hydrolyse un acide dont la valeur pKₛ est inférieure à 4 et un agent tensioactif.

2. Agent selon la revendication 1, caractérisé en ce qu'il contient comme composé libérant de l'acide, un anhydride acide organique, un halogénure d'acide organique ou un composé halogéné inorganique formant un hydracide halogéné lors de l'hydrolyse.

3. Agent selon la revendication 2, caractérisé en ce qu'il contient comme composé libérant un acide, de l'anhydride de l'acide maléique ou du dianhydride de l'acide pyromellitique ou un chlorure d'acide d'un acide carboxylique, phosphonique ou sulfonique.

4. Agent selon la revendication 2, caractérisé en ce qu'il contient comme composé halogéné formant hydracide halogéné, un chlorure ou un oxychlorure de silicium, de titane, d'étain, de phosphore ou de soufre.

5. Agent selon une quelconque des revendications 1 à 4, caractérisé en ce que l'acétal d'acroléine est à chaïne ouverte ou cyclique, ne présente pas de groupes hydroxyle libres et est basé sur un composant alcool monovalent ou bivalent présentant 1 à 6 atomes de carbone.

6. Agent selon la revendication 5, caractérisé en ce qu'il contient du 2-vinyl-1,3-dioxolane comme acétal de l'acroléine.

7. Agent selon une quelconque des revendications 1 à 6, caractérisé en ce que la valeur pKₛ de l'acide libéré est inférieure à 2,5.

8. Agent selon une quelconque des revendications 1 à 7, caractérisé en ce qu'il contient l'acétal d'acroléine et le composé libérant l'acide dans un rapport molaire compris entre 50:1 et 10:1.

9. Agent selon une quelconque des revendications 1 à 8, caractérisé en ce qu'il contient comme agent tensioactif un produit d'éthoxylation, essentiellement exempt de groupes hydroxyle ou pauvre en groupes hydroxyle, d'un alcool gras, d'une amine grasse ou d'un ester monogras ou digras de l'acide glycérique.

10. Agent selon la revendication 1 à 9, caractérisé en ce qu'il contient essentiellement 80 à 98 % en poids d'acétal d'acroléine, 2 à 20 % en poids de composé libérant de l'acide et jusqu'à 5 % en poids d'agent tensioactif.

11. Procédé pour la lutte contre des nuisances microbiennes, végétales et animales à l'aide d'acroléine, caractérisé en ce qu'on met en contact un agent selon une quelconque des revendications 1 à 10, soit directement avec un milieu humide contenant les nuisances à combattre ou en mélangeant l'agent, dans un rapport pondéral de 1:1 à 1:50, avec de l'eau et en introduisant le mélange réactionnel dans le milieu contenant les nuisances à combattre, après un temps de réaction nécessaire à une déacétalisation considérable de l'acétal d'acroléine contenu dans la composition, directement ou après une dilution supplémentaire avec de l'eau.

12. Procédé selon la revendication 11, pour empêcher une croissance excessive d'algues et d'herbes dans des systèmes aquatiques, caractérisé en ce qu'on introduit en continu ou périodiquement dans le système aquatique le mélange réactionnel déacétalisé en une quantité telle que la teneur en acroléine dans ledit système est maintenue dans une plage comprise entre 0,1 et 20 ppm.
